(19) **Europäisches Patentamt**
**European Patent Office**
**Office européen des brevets**

(11) Publication number: **0 034 192**
**A1**

## (12) EUROPEAN PATENT APPLICATION

(21) Application number: **80100754.3**

(22) Date of filing: **14.02.80**

(51) Int. Cl.³: **A 61 F 1/00**

(43) Date of publication of application:
**26.08.81 Bulletin 81/34**

(84) Designated Contracting States:
**AT BE CH DE FR GB IT LU NL SE**

(71) Applicant: **HOWMEDICA INTERNATIONAL, INC.**
**Raheen Industrial Estate**
**Limerick(IE)**

(72) Inventor: **Bedeschi, Paolo**
**21, Via Nicola Fabrizi**
**I-Modena(IT)**

(72) Inventor: **Luppino, Tommaso**
**850, Via Giardini**
**I-Modena(IT)**

(74) Representative: **Hauck, Hans, Dipl.-Ing. et al,**
**Patentanwälte Dipl.-Ing.H.Hauck, Dipl.-Phys.W.Schmitz,**
**Dipl.-Ing.E.Graalfs, Dipl.-Ing.W.Wehnert,**
**Dipl.-Phys.W.Carstens Dr.-Ing.W.Döring Neuer Wall 41**
**D-2000 Hamburg 36(DE)**

(54) **A wrist prosthesis.**

(57) A wrist prosthesis, particularly for the treatment of rheumatoid arthritis which includes a first element (20) fixed to the radius and a second element (21) fixed to the carpal bones. First and second element engage each other through suitable, low-friction bearing surfaces (23, 31).

FIG.1

EP 0 034 192 A1

COMPLETE DOCUMENT

A Wrist Prosthesis          February 13, 1980

The invention refers to a wrist prosthesis.

The treatment of rheumatoid arthritis and other serious disorders of the human wrist has generally been arthrodesis. The
arthrodesis results in a considerable restriction of the
function of the human wrist.

For this reason the invention is to solve the problem to provide
for a wrist prosthesis which enables a more articulated function
than usual surgical methods.

This problem is solved by a first element adapted to be
connected to the radius, said first element having a doubly
concave bearing surface of low friction, and a second element
replacing the scaphoid and lunate bones, said second element
being adapted to be fixed to the associated carpal bones and
being provided with a convex bearing surface co-operating with
the concave bearing surface of the first element.

One of the both elements of the wrist prosthesis according to
the invention can be connected to the radius, while the other
replaces the scaphoid and the lunate bones of the carpal bones
in order to define a counter bearing surface for the bearing
surface of the first element. According to a further embodiment of the invention the first element is plate-shaped and

.../2

made of metal. The plate is preferably designed such that the surface opposite to the bearing surface is extending complementari to the bearing surface, that means is doubly convex. By this, a suitable accommodation to the anatomic data of the extremity of the radius is given so that only a minimal amount is to be resected from the radius before applying the wrist prosthesis according to the invention.

It is to be understood that bio-compatible metal is used, e.g. cobalt-chrome-alloy, stainless steel or titanium.

Suitable means must be provided to fix the first element to the radius. A further embodiment of the invention provides for two fixing pins formed to the first element on the opposite side of the concave bearing surface. The fixing pins are driven into a radius and fixed therein either directly or under use of bone cement (methyl methacrylate). The geometry of the second element is such that anatomic data are substantially copied. According to a further embodiment of the invention the second element consists of bio-compatible plastic material. Suitable plastic material is preferably polyacetal or polyethylene of ultra-high molecular weight.

The second element has to have also means to be fixed to the carpal bones. A further embodiment of the invention provides for two fastening elements of metal formed to the second element on the opposite side thereof with respect to the bearing surface. In case of using plastic material for the second element the fastening elements could be embedded or cast in the second element, respectively. In this connection, a further embodiment of the invention is characterized by two approximately parallel fastening pins which are connected by a transverse web which is embedded in the second element. The fixing pins are preferably of bio-compatible metal as for instance a cobalt-chrome-alloy, stainless steel or titanium. The fixing pins are driven in the respective carpal bones and fixed therin directly or by means of a suitable bone cement.

In order to improve the fixation of the fixing pins of the first and/or second element, a plurality of projections, preferably barbs forming recesses are located in spaced relationship with respect to the longitudinal axis of the pins.

In order to achieve an absolute fixed retention of the second element to the carpal bones, a further embodiment of the invention provides for a plurality of projections, ribs or a toothing on the second element opposite to the bearing surface. These projections resist lateral displacements.

It is understood that the co-operating bearing surfaces of the first and second element have a minimal friction which could be achieved by a respective hardness of the material and surface finish of the bearing surfaces.

One embodiment is to be described in detail according to enclosed drawings.

Fig. 1 is a lateral view of a wrist prosthesis according to the invention.

                              view
Fig. 2 is a bottom plan/of the first element of the wrist prosthesis according to Fig. 1.

Fig. 3 is a section through the illustration according to Fig. 2 along     line 3-3.

Fig. 4 is a section through the illustration according to Fig. 2 along line 4-4.

Fig. 5 shows a detail of the illustration according to Fig. 3.

Fig. 6 is a lateral view of the second element of the wrist prosthesis according to  Fig. 1, partly  in section.

Fig. 7 is a view on the illustration of Fig. 6 from above.

Fig. 8 is a lateral view of the illustration of Fig. 6 in the direction indicated by arrow 8.

Fig. 9 shows the contour of the element according to Fig. 6 at position 9.

Fig. 10 shows the contour of the element according to Fig. 6 at position 10.

Fig. 11 is a section through the element according to Fig. 6 along line 11-11.

Fig. 12 is a section through the element according to Fig. 6 along line 12-12.

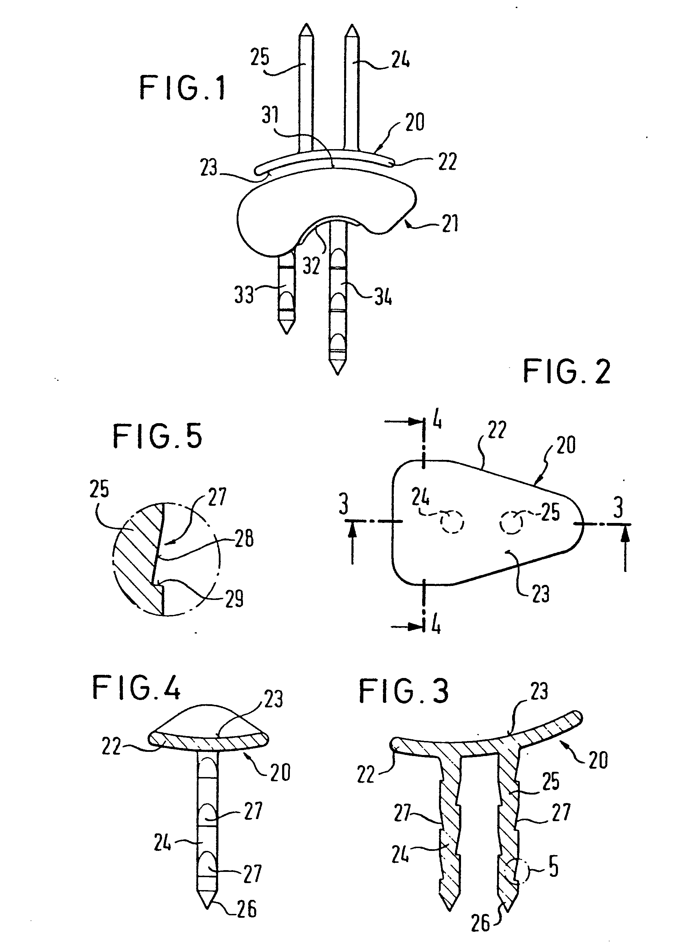

The drawings are not *full* scale, in particular the illustrations according to the Figs. 2 to 12 are enlarged relative to the actual data. The illustration according to Fig. 1, however, substantially corresponds to the anatomic dimensions.

Before the details shown in the drawings are explained it is remarked that each feature per se or in connection with features according to the claims is of essential importance for the invention.

The prosthesis illustrated in Fig. 1 consists of a first element 20 and a second element 21 which are to be explained in detail herebelow.

The first element 20 includes a metallic plate 22 of a cobalt-chrome-alloy, stainless steel or titanium and has approximately equal thickness through its extension, e.g. 2 mm. The shape of plate 22 can be seen in Fig. 2. Simply explained, it is triangular or to be understood as composed of a triangle and rectangle. As can be seen in the Figs. 3 and 4, the plate 22 is doubly curved like a spherical cup so that a doubly concave bearing surface 23 is formed. Accordingly, the opposite side of the plate 22 is complementarily formed. On this side, two fixing pins 24, 25 spaced from each other and extending in parallel are formed to the plate, the fixing pins lying on the longitudinal axis of the plate. The fixing pins 24, 25 are sharpened at their extremities at 26 and have recesses 27 on diametrically opposite sides, the recesses 27 of the fixing pins 24, 25 provide for a barb effect.

In Fig. 5 such a recess 27 is illustrated as encircled at arrow 5 in Fig. 3. The recess is defined by two intersecting surfaces 28 and 29, the first of the surfaces is at an angle of $10^{o}$ with respect to the longitudinal axis of the fixing pin 25, while the surface 29 extends at an angle of $5^{o}$ with respect to the perpendicular to the longitudinal axis. The fixing pins 24, 25 are driven into the radius, and it can be seen that the recesses 27 cause a minimal resistance to such displacement in contrast to extraction.

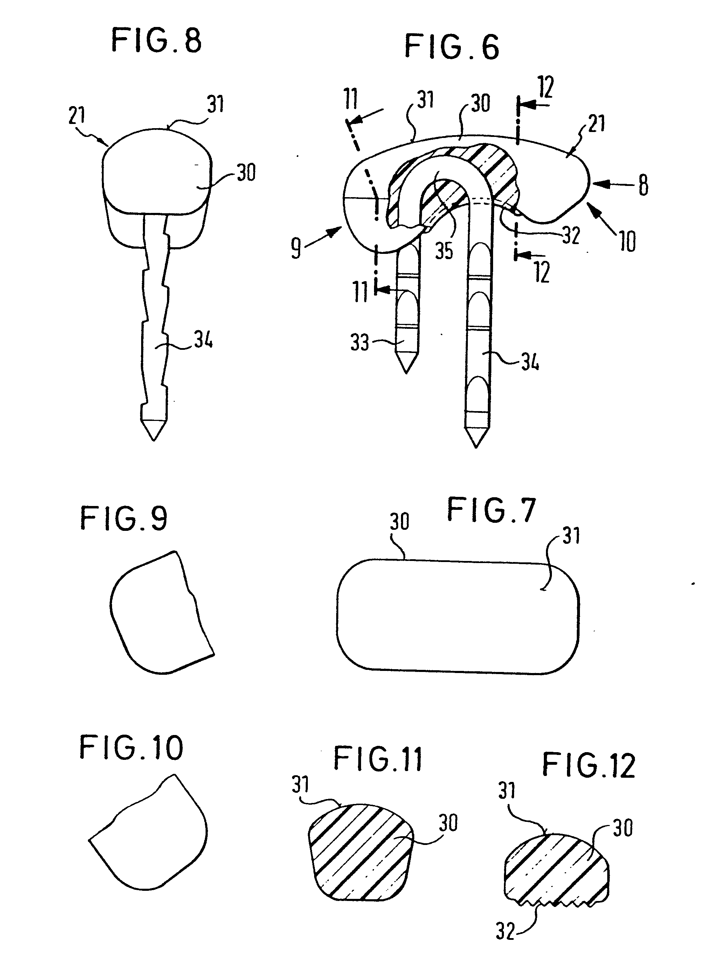

The second element includes a body 30 of banana-like shape. In its geometry it accommodates the shape of the scaphoid and lunate bones of the human wrist. The body 30 is made of bio-compatible plastic material, e.g. polyacetal or polyethylene of ultra-high molecular weight. If seen from above, the body 30 has approximately a rectangular shape as can be seen in Fig. 7. The contour at the positions 9 and 10 of the body 30 can be seen in the Figs. 9 and 10. The upper surface defines
a bearing surface 31 which    accommodates the curvature of
the bearing surface 23 of the element 20, however, the convex
curvature of the bearing surface 31 is considerably larger
than the corresponding curvature of the element 20 or the
bearing surface 23, respectively. The cross sectional shape
of the body 30 can be seen in the Figs. 11 and 12, Fig. 12
is showing that the body 30 has a plurality of parallel ribs
32 triangular in cross section and oppositely located to
the bearing surface 38 in a mediate portion wherein it abuts
on the capitate of the human wrist.

The second element 21 includes further two fixing pins 33, 34
parallel to and spaced from each other and protruding from the
body 30 opposite to the bearing surface 31. The design of
the fixing pins 33, 34 resembles the design of the fixing
pins 24, 25 so that it is refrained from going into further
detail. However, it is mentioned that the fixing pin 33 is
considerably shorter than the fixing pin 34. The fixing
pins 33, 34 are driven into the respective carpal bones and
fixed therein either directly or by means of bone cement.
The fixing pins 33, 34 are integrally connected to each other
by an arcuate connection web 35 which is embedded in the body
30 made of plastic material.

Upon application, the fixing pins 24, 25 of the first element 20
are driven into the radius after the forward extremity is
treated. The fixing pins 24, 25 are secured to the radius with or without use of bone cement. The fixing pins 33, 34 are driven into the respective carpal bones, that means into the capitate and the trapezoideum bones and pass into the metacarpal bones. The fixing pins are secured with or without the use of bone cement. The tooth-like ribs engage the respective carpal bones, particularly the capitate, and resist forces transverse to the axis of the fixing pins 33, 34.

Claims:

1. A wrist prosthesis, characterized by a first element (20) adapted to be fixed to the radius, said first element (20) having a doubly concave bearing surface (23) of low friction, and by a second element (21) replacing scaphoid and lunate bones, said second element being adapted to be fixed to the associated carpal bones and provided with a convex bearing surface (31) co-operating with the concave bearing surface (23) of the first element (20).

2. A wrist prosthesis according to claim 1, characterized in that the first element (20) includes a plate (22) of metal.

3. A wrist prosthesis according to claim 1 or 2, characterized in that two fixing pins (24, 25) are integrally moulded to the first element (20) on the side thereof located opposite the concave bearing surface (23).

4. A wrist prosthesis according to one of the claims 1 to 3, characterized in that the second element (21) consists of a bio-compatible plastic material.

5. A wrist prosthesis according to one of the claims 1 to 4, characterized in that fixing elements (33, 34) made of metal are embedded in or integrally cast with the second element (21) and extending from the second element opposite into the bearing surface (31).

6. A wrist prosthesis according to claim 5, characterized in that two approximately parallel fixing pins (33, 34) are provided which are connected by a web (35) which is embedded in or integrally cast with the second element (21).

7. A wrist prosthesis according to claim 3 or 6, characterized in that the fixing pins (24, 25; 33, 34) have recesses (27) located in spaced relationship with respect to the longitudinal axis of the pins, said recesses forming barbs.

8. A wrist prosthesis according to one of the claims 1 to 7, characterized in that the second element (21) has a plurality of projections, preferably ribs or a toothing located opposite to the bearing surface (31).

FIG.1

FIG.2

FIG.5

FIG.4

FIG.3

## FIG.8

## FIG.6

## FIG.9

## FIG.7

## FIG.10

## FIG.11

## FIG.12

**European Patent Office**

## EUROPEAN SEARCH REPORT

| | DOCUMENTS CONSIDERED TO BE RELEVANT | | CLASSIFICATION OF THE APPLICATION (Int. Cl.3) |
|---|---|---|---|
| Category | Citation of document with indication, where appropriate, of relevant passages | Relevant to claim | |
| | US - A - 4 178 640 (U.H. BUECHLER et al.) <br> * claim 1; fig. 1 * <br> -- | 1,5, 6,7 | A 61 F 1/00 |
| | US - A - 4 106 128 (A.S. GREENWALD et al.) <br> * claim 1; fig. 8 * <br> -- | 1 | |
| | US - A - 4 063 314 (A.G. LODA) <br> * claim 1; fig. 1 * <br> -- | 1,5,6 | |
| | US - A - 4 040 130 (G.R. LAURE) <br> * claim 1; fig. 1 * <br> -- | 1,5,6 | TECHNICAL FIELDS SEARCHED (Int.Cl.3) |
| | US - A - 4 003 096 (O. FREY) <br> * claim 1 * <br> -- | 1 | A 61 F 1/00 |
| | US - A - 3 909 853 (W.M. LENNOX) <br> * claim 1 * <br> -- | 1,7 | |
| | US - A - 3 837 008 (A. BAHLER et al.) <br> * claim 1 * <br> -- | 1,7 | |
| | GB - A - 2 007 099 (R.S. HAMAS) <br> * claim 1; fig. 5 * <br> -- | 1,8 | CATEGORY OF CITED DOCUMENTS |
| A | US - A - 3 774 244 (P.S. WALKER) <br> * entire document * <br> -- | 1,2,4 | X: particularly relevant <br> A: technological background <br> O: non-written disclosure <br> P: intermediate document <br> T: theory or principle underlying the invention |
| A | US - A - 3 748 662 (A.J. HELFET) <br> * entire document * <br> ---- | 1,2, 4,7 | E: conflicting application <br> D: document cited in the application <br> L: citation for other reasons |

&: member of the same patent family, corresponding document

| | The present search report has been drawn up for all claims | | |
|---|---|---|---|
| Place of search <br> Berlin | Date of completion of the search <br> 13-10-1980 | Examiner <br> KANAL | |